# NEW EUROPEAN PATENT SPECIFICATION

(11) **EP 0 556 345 B2**
(45) Date of publication and mention of the opposition decision: **12.10.2005**
(45) Mention of the grant of the patent: 02.01.1997
(21) Application number: 92904720.7
(22) Date of filing: 31.10.1991
(51) Int. Cl.: C12N 15/63, C12N 15/86, C12N 15/67, C12N 15/12, C12N 7/01, C12N 9/72, C12N 5/10

(54) **RETROVIRAL VECTORS USEFUL FOR GENE THERAPY**
NÜTZLICHE RETROVIRALE VEKTOREN FÜR DIE GENTHERAPIE
VECTEURS DE RETROVIRUS EFFICACES EN THERAPIE GENIQUE

(30) Priority: 31.10.1990 US 607252
(43) Date of publication of application: 25.08.1993
(73) Proprietor: CELL GENESYS, INC., Foster City, CA 94404 (US); WHITEHEAD INSTITUTE FOR BIOMEDICAL RESEARCH, Cambridge, MA 02142 (US)
(72) Inventor: GUILD, Braydon, C., Concord, MA 01742 (US); RAFIELD, Lori, F., San Francisco, CA 94123 (US); COHEN, Lawrence, K., Oakland, CA 94611 (US); ROBBINS, Paul, Mt. Zebanon, PA 15216 (US); MULLIGAN, Richard, C., Cambridge, MA 02138 (US)
(74) Representative: Vossius, Volker
(86) International application number: PCT/US1991/008121
(87) International publication number: WO 1992/007943

(56) References cited:
- WO-A-89/07136
- WO-A-90/02806
- US-A- 4 963 481
- BLOOD, vol. 75, 01 March 1990; ISRAEL et al., pp. 1074-1080
- NUCLEIC ACIDS RESEARCH, vol. 18, no. 12, 25 June 1990; MORGENSTERN et al., pp. 3587-3596
- JOURNAL OF VIROLOGY, vol. 50, no. 1, April 1984; EMERMAN et al., pp. 42-49
- JOURNAL OF VIROLOGY, vol. 61, no. 5, May 1987; BENDER et al., pp. 1639-1646
- MOLECULAR & CELLULAR BIOLOGY, vol. 7, no. 10, October 1987; LIM et al., pp. 3459-3465
- PROCEEDINGS OF THE NATL. ACADEMY OF SCIENCES USA, vol. 85, June 1988, Washington, DC (US); WILSON et al., pp. 4421-4425

## Description

The present invention is directed to a novel retroviral vector capable of being used in somatic gene therapy The retroviral vector includes an insertion site for the genes of interest and are capable of expressing controlled levels of the protein derived from the genes of interest in a wide variety of transfected cell types. The novel retroviral vector of the invention lacks a selectable marker, thus rendering it suitable for human somatic therapy in the treatment of a variety of disease states without the coproduction of an marker gene products, such as antibiotic resistance peptides. The retroviral vector of the invention is especially suited for use in certain packaging cell lines.

### Background

Numerous methods exist for genetically engineering mammallan cells. There is great interest in genetically engineering mammalian cells for several reasons including the need to produce large quantities of various polypeptides and the need to correct various genetic defects in the cells. The methods differed dramatically from one another with respect to such factors as efficiency, level of expression of foreign genes, and the efficiency of the entire genetic engineering process.

One method of genetically engineering mammalian cells that has proven to be particularly useful is by means of retroviral vectors. Retroviral vectors and their uses are described in many publications including Mann, et al., Cell 33:153-159 (1983) and Cone and Mulligan, Proc. Natl. Acad. Sci. USA 81:6349-6353 (1984). Retroviral vectors are produced by genetically manipulating retroviruses.

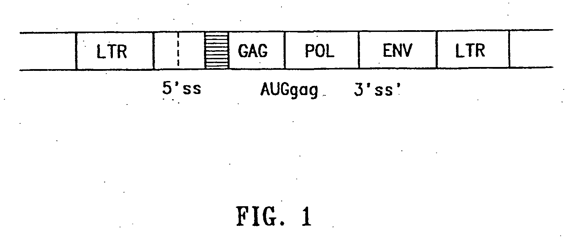

Retroviruses are RNA viruses; that is, the viral genome is RNA. This genomic RNA is, however, reverse transcribed into a DNA copy which is integrated stably and efficiently into the chromosomal DNA of transduced cells. This stably integrated DNA copy is referred to as a provirus and is inherited by daughter cells as any other gene. As shown in Figure 1, the wild type retroviral genome and the proviral DNA have three genes: the gag, the pol and the env genes, which are flanked by two long terminal repeat (LTR) sequences. The gag gene encodes the internal structural (nucleocapsid) proteins; the pol gene encodes the RNA directed DNA polymerase (reverse transcriptase); and the env gene encodes viral envelope glycoproteins. The 5' and 3' LTRs serve to promote transcription and polyadenylation of virion RNAs.

Adjacent to the 5' LTR are sequences necessary for reverse transcription of the genome (the tRNA primer binding site) and for efficient encapsidation of viral RNA into parties (the Psi site). Mulligan, R.C., in: Experimental Manipulation of Gene Expression, M. Inouye (ed). 155-173 (1983); Mann, R., et al., Cell, 33:153-159 (1983); Cone, R.D. and R.C. Mulligan, Proceedings of the National Academy of Sciences, U.S.A., 81: 6349-6353 (1984).

If the sequences necessary for encapsidation (or packaging of retroviral RNA into infectious virions) are missing from the viral genome, the result is a cis acting defect which prevents encapsidation of genomic RNA. However, the resulting mutant is still capable of directing the synthesis of all virion proteins. Mulligan and coworkers have described retroviral genomes from which the Psi sequence has been deleted, as well as cell lines containing the mutant genome stably integrated into the chromosome. Mulligan, R.C., In Experimental Manipulation of Gene Expression, M. Inouye (ad), 155-173 (1983); Mann, R. et al., Cell, 33:153-159 (1983); Cone, R.D. and R.C. Mulligan, Proceedings of the National Academy of Sciences, U.S.A., 81:6349-6353 (1984). Additional details on available retrovirus vectors and their uses can be found in patents and patent publications Including European Patent Application EPA 0 178 220, U.S. Patent 4,405,712, Gilboa, Biotechniques 4: 504-512 (1986) (which describes the N2 retroviral vector). The teachings of these patents and publications are incorporated herein by reference.

Retroviral vectors are particularly useful for modifying mammalian cells because of the high efficiency with which the retroviral vectors "infect" target cells and integrate into the target cell genome. Additionally, retroviral vectors are highly useful because the vectors may be based on retroviruses that are capable of infecting mammalian cells from a wide variety of species and tissues.

The ability of retroviral vectors to insert into the genome of mammalian cells have made them particularly promising candidates for use in the genetic therapy of genetic diseases in humans and animals. Genetic therapy typically involves (1) adding new genetic material to patient call in vivo, or (2) removing patient cells from the body, adding new genetic material to the cells and reintroducing them into the body, i.e., in vitro gene therapy. Discussions of how to perform gene therapy in a variety of cells using retroviral vectors can be found, for example, in U.S. Patent Nos. 4,868,116, issued September 19, 1989, and 4,980,286, issued December 25, 1990 (epithelial cells), WO89/07136 published August 10, 1989 (hepatocyte cells), EP 378,576 published July 25, 1990 (fibroblast cells), and WO89/05345 published June 15, 1989 and WO/90/06997, published June 28, 1990 (endothelial cells), the disclosures of which are incorporated herein by reference.

In order to be useful for the various techniques of gene therapy, suitable retroviral vectors require special characteristics that have not hitherto been available. A primary source of the need for these special requirements of the vector for use in the in vivo genetic manipulation of patient cells in gene therapy is because It is usually not feasible to use retroviral vectors that require a selection for integration of the vector into the genome of "patient" cells. For example, typical retroviral vectors, e.g., MSV DHFR-NEO described in Williams, et al., Nature 310:476-480 (1984), uses neomycin resistance as a suitable marker for detecting genetically modified cells. Thus, with such neomycin resistant retroviral vectors, patients would be required to be exposed to high levels of neomycin in order to effect genetic repair of cells through in vivo gene therapy. Moreover, in both in vivo and in vitro gene therapy it may be undesirable to produce the gene product of the marker gene in cells undergoing human gene somatic therapy. For example, there is no therapeutic reason to produce large levels of neomycin phosphotransferase in blood calls undergoing hemoglobin gene replacement for curing a thalassemia. Therefore, it would be desirable to develop retroviral vectors that integrate efficiently into the genome, express desired levels of the gene product of interest, and are produced in high titers without the coproduction or expression of marker products such as antibiotic resistance peptides.

### Summary of the Invention

The present invention is directed to a novel retroviral vector capable of being used in somatic gene therapy. The retroviral vector includes an insertion site for the genes of interest and are capable of expressing desired levels of the protein derived from the gene of interest in a wide variety of transfected cell types.

In one aspect of the invention there is provided a retroviral vector comprising in operable combination, a 5' LTA and a 3' LTR derived from a retrovirus of interest i a psi site; an insertion site for a gene of interest, and wherein at least one of the gag, env or pol genes in the vector are incomplete or defective. The vector preferably contains a splice donor site and a splice acceptor site, wherein the splice acceptor site is located upstream from the site where the gene of interest is inserted. Also, the vector contains a gag transcriptional promoter functionally positioned such that a transcript of a nucleotide sequence inserted into the insertion site is produced, and wherein the transcript comprises the gag 5' untranslated region. The vector of the invention is lacking a selectable marker, thus, rendering it more dasireable in human somatic gene therapy because a marker gene product, such as an antibiotic drug marker will not be co-produced or co-expressed.

The gene of interest that is incorporated in the vector of the invention may be any gene which produces a hormone, an enzyme, a receptor or a drug(s) of interest.

The retroviral vector is most suitably used in combination with certain packaging cells, as herein defined, which in turn may be used in a wide variety of cell types for human or animal somatic gene th erapy.

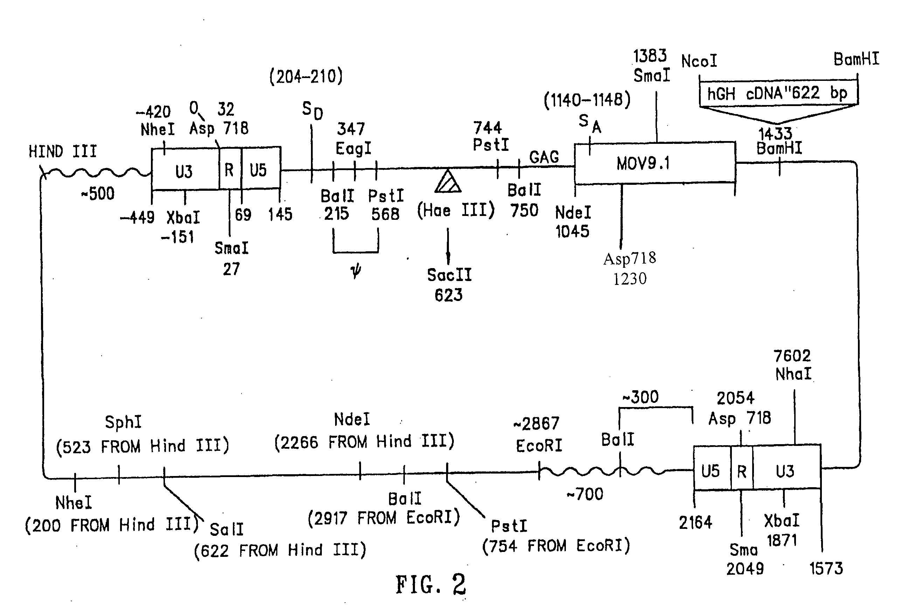

The hGH insert, retroviral vector of the invention is identified herein as "MFG", as depicted in Figure 2 without, and the plasmid containing it. The plasmid MFG has the identifying characteristics of ATCC No. 68,754.

The subject invention also includes the retroviral vector that has a gene for expression inserted into the site for gene expression. Specific examples include MFG vectors containing the genes for human factor VIII or tPA inserted into the site for expression of these useful products.

### Description of Drawings

Figure 1 is a schematic representation of a wild type murine leukemia virus (retroviral) genome.
Figure 2 is a schematic representation of the retroviral vector MFG with an hGH insert.
Figure 3 is a histogram showing the potency after implantation into dogs of synthetic grafts lined with endothelial cells genetically augmented to express tPA.

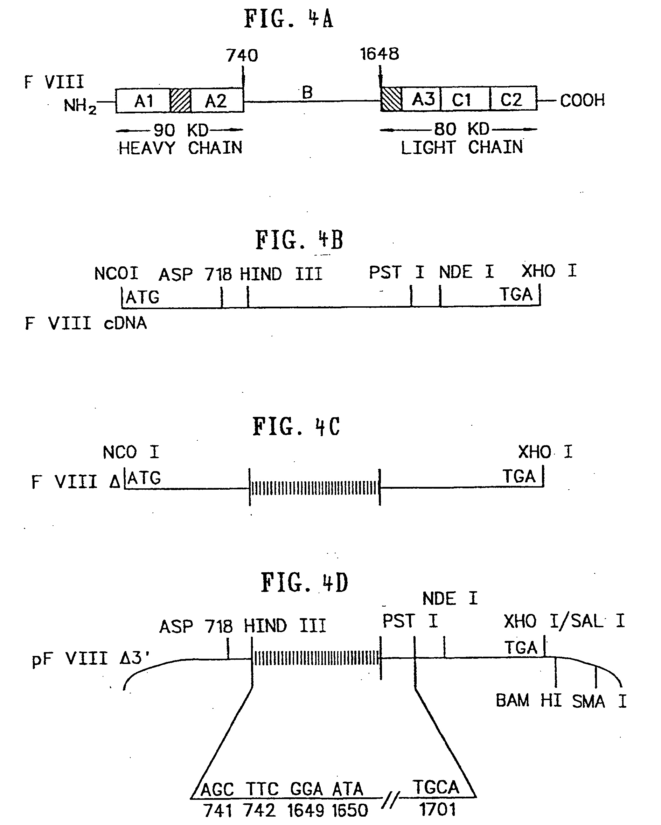

Figure 4a is a diagram of the factor VIII polypeptide. Figure 4b is a diagram of the factor VIII cDNA showing the restriction enzyme sites used in the various constructs to generate the retroviral vector. Figure 4c is a diagram of the deletion derivative of the factor VIII cDNA inserted into the retroviral vector with the deleted region shown as vertical lines. Figure 4d is an expanded diagram of the B domain deletion between the Hind III and Pst I sites. The nucleotide sequence at the junction of the heavy chain and light chain is denoted above the line and the corresponding amino acid numbers are denoted below the line.
Figure 5 is a diagram of the assembled final retroviral vector, MFG-factor VIII.
Figures 6(a) and 6(b) represents a schematic diagram of the construction of the MFG vector of the invention.
Figure 7 is a schematic representation of the modification of the tPA gene, the oligonucleotides used to facilitate the modification and the insertion of the modified tPA gene into the MFG vector.

### Description of Specific Embodiments

The subject invention provides a novel retroviral vector as claimed. The retroviral vector provided for contain (1) 5' and 3' LTRs derived from a retrovirus of interest, the preferred retrovirus source for the LTRs is the Moloney murine leukemia virus, and (2) an insertion site for a gene of interest. The retrovirus vector of the subject invention does not contain either a complete gag, env, or pol gene, so that the retroviral vector is incapable of independent replication in target cells. The retroviral vector contains a portion of the gag coding sequence, preferably the partial gag coding sequence comprises a splice donor site. The splice acceptor site is located closest to, and upstream from, the insertion site for the gene of interest. In a particularly preferred embodiment of the subject vector, the gag transcriptional promoter is positioned such that a transcript initiated from the gag promoter contains untranslated 5' gag sequence and transcript produced from nucleic acid sequence inserted into the insertion site in the vector. The vector of interest does not contain selectable markers. The embodiment of such a vector is the vector designated as "MFG".

Another aspect of the subject invention is to provide for retrovirus vector constructions containing genes for expression inserted into the insertion site in the retrovirus vector. Genes for insertion into the subject retrovirus vector include any at a variety of hormones, enzymes, receptors or other drugs. The subject invention specifically provides for the genetic constructions consisting of tPA and Factor VIII inserted (individually) into the insertion, i.e., cloning site of MFG.

The wild type retroviral genome has been modified by Cone and Mulligan, supra for use as a vector capable of introducing new genes into cells. The gag, the pol and the env genes have all been removed and a DNA segment encoding the neo gene has been inserted in their place. The neo gene serves as a dominant selectable marker. The retroviral sequence which remains part of the recombinant genome includes the LTRs, the tRNA binding site and the Psi packaging site, Cepko, C. et al., Cell, 37:1053-1062 (1984).

In addition to teaching the retroviral vector containing sites for insertion of foreign genes for expression, the subject invention also provides for genetic constructions in which the retroviral vector contains genes inserted into the site for insertion i.e., foreign genes or genes for expression. Foreign genes for inclusion in the vector of the subject invention may encode a variety of proteins. Proteins of interest include various hormones, growth factors, enzymes, lymphokines, cytokines, receptors and the like. The term "foreign genes" includes nucleic acid sequences endogenous to cells into which the retrovirus vector containing the foreign gene may be inserted. Of particular interest for use as genes for expression are those genes encoding polypeptides either absent, produced in diminished quantities, or produced in mutant form in individuals suffering from a genetic disease. Additionally, it is of interest to use foreign genes encoding polypeptides for secretion from the target cell so as to provide for a systemic effect by the protein encoded by the foreign gene. Specific foreign genes of interest include those encoding hemoglobin, interleukin-1, interleukin-2, interleukin-3, interleukin-4, interleukin-5, interleukin-6, interleukin-7, interleukin-8, interleukin-9, interleukin-10, interleukin-11, etc., GM-CSF, G-CSF, M-CSF, human growth factor, insulin, factor VIII, factor IX, tPA, LDL receptors, tumor necrosis factor, PDGF, EGF, NGF, IL-1ra, EPO, β-globin and the like, as well as biologically active muteins of these proteins. Genes for expression for insertion into the retroviral vector may be from a variety of species; however, preferred species sources for genes of interest are those species into which the retroviral vector containing the foreign gene of interest is to be inserted.

The retroviral vector of the subject invention is typically used by transfecting the nucleic acid sequences into packaging cell lines. Packaging cell lines contain viral gene functions that have been deleted from the retrovirus in the course of converting it to a vector. Thus transfecting the retroviral vector of the subject invention, either with or without genes for expression inserted into the vector insertion site, into packaging cell lines results in the production of infectious virus particles containing the desired genetic construction. Ideally, packaging cell lines are capable of producing a high titer of recombinant retrovirus. Preferred packaging cell lines for use with the genetic constructions of the subject are Psi2, Psi-Am, Psi CRIP, and Psi CRE. Psi2 is particularly preferred for use with the retroviral vector MFG.

The Psi 2 cell line described by Mulligan and coworkere was created by transfecting NIH 3TS cells with pMOV-Psi, which is an ecotropic Moloney murine leukemia virus (Mo-MuLV) clone. pMOVPsi expresses all the viral gene products but lacks the Psi sequence, which is necessary for encapsidation of the viral genome. pMOV-Psi- expresses an acotropic viral envelope glycoprotein which recognizes a receptor present only on mouse (and closely related rodent) cells.

Another cell line is the Psi am line, which is a Psi-2-like packaging cell line. This Psi-am cell line contains a modified pMOV-Psi-genome, in which the acotropic envelope glycoprotein has been replaced with envelope sequences derived from the amphotropic virus 4070A. Hartley, J.W. and W.P. Rowe, Journal of Virology, 19: 19-25 (1976). As a result, they are useful for production of recombinant virus with amphotropic host range. The retrovirus used to make the Psi am cell line has a very broad mammalian host range (an amphotropic host range) and can be used to infect human cells. If the recombinant genome has the Psi packaging sequence, the Psi-am cell line is capable of packaging recombinant retroviral genomes into infectious retroviral particles. Cone, R. and Mulligan, RC. Proceedings of the National Academy of Sciences. USA, 81:6349-6353 (1984).

Two other packaging cell lines are known as Psi CRIP and Psi CRE. These cell lines have been shown to be useful to isolate clones that stably produce high titers of recombinant retroviruses with amphotropic and ecotropic host ranges, respectively. These cell lines are dascribed in Danos, 0, and R.C. Mulligan, Proceedings of the National Academy of Sciences, USA, 85: 6460-6464 (1988). Psi CRIP and Psi CRE have been deposited at the American Type Culture Collection, Rockville, MD, under Accession Nos. CRL 9808 and CRL 9807, respectively, under the terms of the Budapest Treaty.

The retroviral vector or the invention may be used in a wide variety of cell types, including without limitation, epithelial cells, fibroblast cells, hepatocyte cells, endothelial cells, myoblast cells, astrocyte cells, lymphocyte cells, mesenterial cells, and the like. Of particular interest are the cell types disclosed in the following patents and patent publications U.S. Patent Nos. 4,868,116, issued September 19, 1989, and 4,980,286, issued December 25, 1990 (epithelial cells), PCT/US89/00422, WO89/07136 published August 10, 1989 (hepatocyte cells), EP 378,576 published July 25, 1990 (fibroblast cells), and PCT/US88/04383, WO89/05345 published June 15, 1989 and WO/80/06997, published June 28, 1990 (endothelial cells).

The vector of the subject invention finds a variety of uses in the treatment of various medical conditions including, without limitation cancer, genetically based diseases, cardiopulmonary diseases, endocrinological diseases, and the like.

The present invention will now be illustrated by the following examples, which are not intended to be limiting in any way.

In Examples 1(a)-(c) there is described the precursor for constructing the MFG vector of the invention.

### Example Ia

### Construction of Vectors

pMOV. (pMOVPsi) was constructed as follows: Three purified DNA fragments were ligated together to construct pMDV Psi. The first was obtained by digesting pMOV Psi+ with Xho I to completion, followed by partial digestion with EcoRI. Chumakov, I. et al., Journal of Virology, 42:1088-1098 (1982). The fragment extending from the Xho I site at 2.0 U in MuLV, through the 3' LTR, 3' mouse flanking sequence, all of pBR322, and ending at the EcoRI site was purified from an agarose gel after electrophoretic separation. Vogeistein, B. and D. Gillespie, Proceedings of the National Academy of Sciences, USA, 761:615-619 (1979). The second fragment was obtained by digestion of pMOV Psi+ with Bal I to completion followed by purification of the fragment extending from the Bal I site in pBR322 through 5' mouse flanking sequence and 5' LTR to the Bal I site located at 0.7 U of MuLV. HindIII linkers (Collaborative Research) were then blunt-ligated to this fragment with T4 DNA ligase, and the fragment was digested with excase HindIII and EcoRI. The LTR- containing fragment was purified from an agarose gel after electrophoretic separation. The third fragment present in the final ligation reaction was obtained from pSV2gag/pol where the gag/pol region of MuLV had been subcloned into pSV2. Mulligan, R.C. and P. Berg, Science, 209:1422-1427 (1980). pSV2- gag/pol was digested to completion with Xho I and HindIII and the fragment extending from the HindIII site (changed from the Pst I site at 1.0 U of MuLV) to the Xho I site at 2.0 of MuLV was purified from an agarose gel following electrophoretic separation. These three DNA fragments were then mixed in equimolar amounts at a total DNA concentration of 50 ug/ml in ligase buffer (50 mM Tris-HCl [pH 7.8], 10 mM MgCl₂, 20 mM dithiothreitol, 1.0 mM ATP, 50 ug/ml bovine serum albumin) and incubated with T4 DNA ligase for 18 hr. at 15 °C. E. coli HB101 was translected with the ligated DNA, and ampicillin resistant transfectants were obtained. The plasmid DNA obtained from a number of transformants was screened for the desired structure by digestion with appropriate restriction endonucleases and electrophoresis through agarose gels. Davis, R.W. et al., Methods in Enzymology, 65:404-411 (1980).

Cell lines containing the Psi mutant stably integrated into the chromosome were made by cotransfection of pMOV-Psi and pSV2gpt, a SV40 hybrid vector capable of XG PRT expression. Mulligan, R.C. and P. Berg. Science, 209:1422-1427 (1980). Cells from gpt+ colonies obtained in this way were cloned and established into three lines: Psi-1, Psi-2, and Psi-3.

### Example 1(b)

pLJ. The characteristics of this vector have been described in Korman, A.J. et al., Proceedings of the National Academy of Sciences, USA, 84:2150 (1987). This vector is capable of expressing two genes: the gene of interest and a dominant selectable marker, such as the neo gene. The gene of interest is cloned in direct orientation into a BamHI/SmaI/SalI cloning site just distal to the 5' LTR, while, the neo gene is placed distal to an internal promoter (from SV40) which is located 3' of the cloning site. Transcription from pLJ is initiated at two sites: 1) the 5' LTR, which is responsible for expression ot the gene of interest and 2) the internal SV40 promoter, which is responsible for expression of the neo gene.

In pLJ, the genetic material of interest is inserted just following the 5' LTR. Expression of this genetic material is transcribed from the LTR and expression of the neo gene is transcribed from an internal SV40 promoter.

### Example I(c)

pEm. In this simple vector, the entire coding sequence for gag, pol and env of the wild type virus is replaced with the gene of interest, which is the only gene expressed. The components of the pEm vector are described below. The 5' flanking sequence, 5' LTR and 400 bp of contiguous sequence (up to the BamHI site) is from pZIP. The 3' flanking sequence and LTR are also from pZIP; however, the Clal site 150 bp upstream from the 3' LTR has been ligated with synthetic BamHI linkers and forms the other half of the BamHI cloning site present in the vector. The HindIII/EcoRI fragment of pBR322 forms the plasmid backbone. This vector is derived from sequences cloned from a strain of Moloney Murine Leukemia virus. An analogous vector has been constructed from sequences derived from the myeloproliferative sarcoma virus.

### Example II

### Construction of the MFG Vector

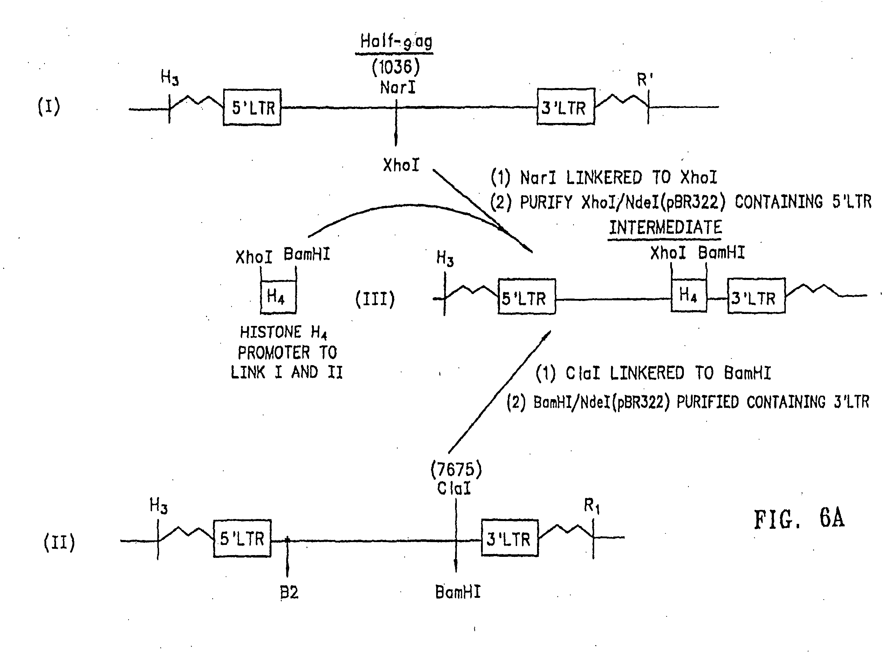

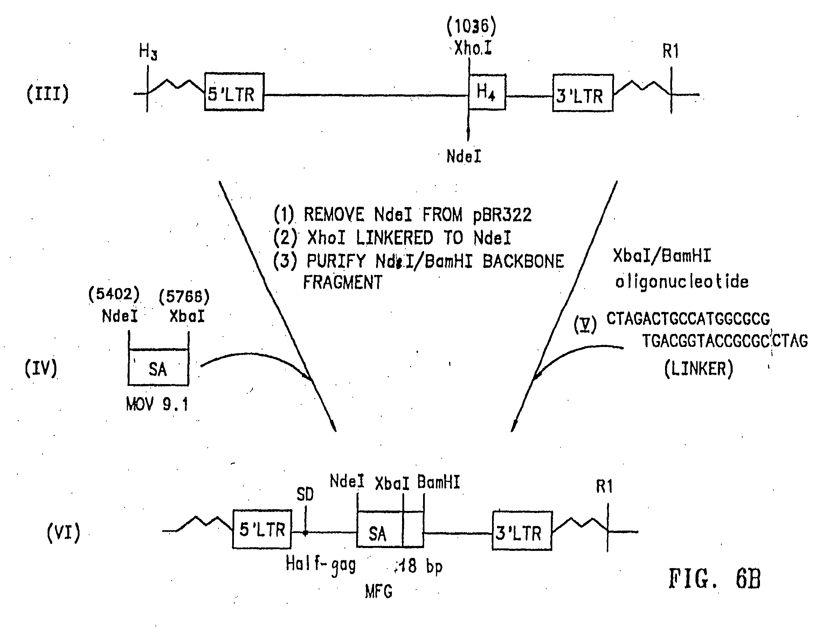

The MFG vector having the identifying characteristics of ATCC accession No. 68754 is derived from the pEm vector but contains 1038 base pairs of the gag sequence from MMLV to increase the encapsulation of recombinant genomes in the packaging cell lines, and 350 base pairs derived from MOV-9 which contains the splice acceptor sequence. An 18 base pair oligonucleotide containing NcoI and BamHI sites directly follows the MOV-9 sequence and allows for the convenient insertion of genes with compatible sites. The MMLV LTR controls transcription and the resulting mRNA contains the authentic 5' untranslated region of the native gag transcript followed directly by the open reading frame of the inserted gene. A detailed map of MFG is provided in Figure 2. Details for the construction of MFG are provided in Figures 6(a) and 6(b).

MFG was constructed by ligating the 5' LTR containing XhoI/NdeI fragment of the half-gag retroviral vector (half-gag is described in Bender, et al., (1987) J. Virol. 6I:1639-1646) to an XhoI/BamHI H4 histone promoter fragment. Retroviral vector pEm was digested with NdeI and BamHI, and the 3' LTR containing fragment was ligated to the half-gag fragment already ligated to the H4 fragment so as to produce an intermediate retrovirus vector containing 2 LTRs in the proper orientation and also containing the H4 fragment within the viral portion of the vector. The intermediate vector was then linearized by digestion with NdeI and the NdeI site in the pBR322 portion of the vector was filled in by polymerase and destroyed by ligation. The vector was subsequently digested with XhoI and the XhoI site was joined to an NdeI linker. The vector was subsequently cleaved with BamHI and the large fragment containing both LTRs and the pBR322 sequence) was purified.

A linker having XhoI and BamHI sites and having the following sequence: 
CTAGACTGCCATGGCGCG
TGACGGTACCGCGCCTAG
 was synthesized and ligated to both the BamHI site on the cleared intermediate vector and an NdeI/XbaI fragment from pMOV9 [containing a splice acceptor site next to the NdeI edge] so as to form a circular vector, MFG as illustrated in Figures 2 and 6(a) to 6(b). The plasmid containing vector MFG has been deposited with the American Type Culture Collection and it has accession number 68,754.

### EXAMPLE IV Increased Expression of tPA by Genetically Modified Canine and Human Endothelial Cells

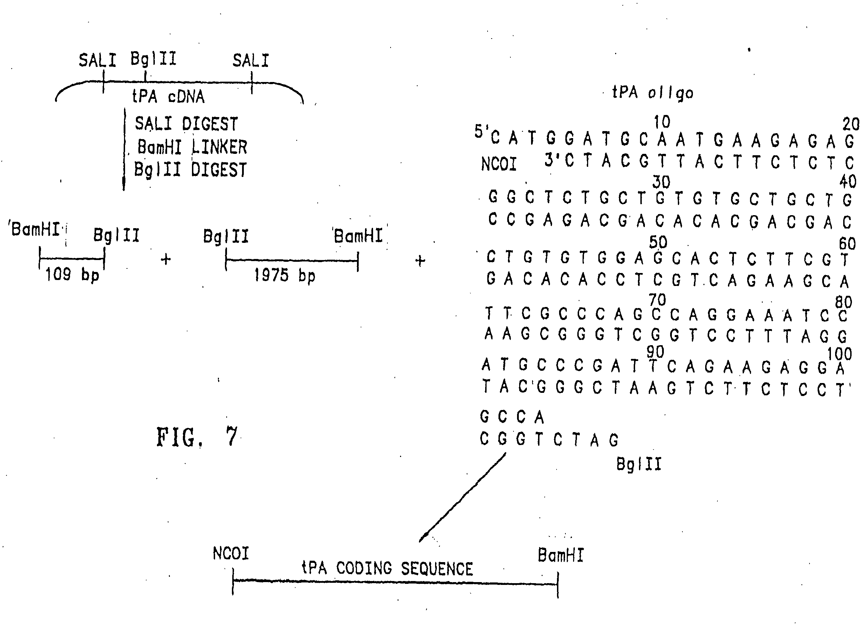

Tissue plasminogen activator (tPA) is a protein normally secreted by endothelial cells that promotes fibrinolysis of blood clots. Recombinant retroviral vectors encoding human tPA were constructed and used to transduce canine endothelial cells in order to demonstrate the enhanced delivery of a therapeutically relevant protein from transduced endothelial cells. The modifications of the tPA gene for cloning into the recombinant retroviral vectors are shown in Figure 7. The coding sequences of human uterine tPA were contained within a Sal I DNA fragment of a pUC-based plasmid obtained from Integrated Genetics Inc. Framingham MA. The Sal I fragment was derived by placing Sal I linkers at the SFaN I site at base pair 6 and the Bgl II site at base pair 2090 of the original cDNA. The coding sequences extends from base pair 13 to base pair 1699.

From this original clone a fragment that could be cloned directly into the MFG vector described in the body of this patent was derived. The Sal I fragment was first converted to a Bam HI fragment by the addition of synthetic Bam HI linkers and then digested with the restriction enzyme Bgl II to yield a 109 base pair BamHI to Bgl II fragment and a 1975 base pair Bgl II to Bam HI fragment. To recreate the missing 100 base pairs of tPA coding sequences and the translational start codon, two 104 base pair oligonucleotides were chemically synthesized and annealed to create a fragment with an Nco I site at the 5' end and a Bgl II site at the 3' end. This oligonucleotide was ligated onto the Bgl II site of the partial 1975 base pair tPA gene to create a 2079 base pair tPA gene with the identical coding sequence of the original molecule, but which can be easily obtained as an Nco I to Bam HI fragment. It was inserted directly into the MFG vector (the resulting vector was given ATCC accession number 68727). These manipulations were performed by standard molecular biological techniques (Molecular Cloning -A laboratory Manual, T. Maniatis, E.F. Frisch, and J. Sambrook), and are diagrammed in Figure 2 and 6a and 6b.

Cell lines producing recombinant virus encoding MFG-tPA were made from the Psi packaging cell line of Danos and Mulligan capable of producing recombinant retrovirus of amphotrophic host range [Proc. Natl. Acad. Sci. U.S.A. 85:6460 (1988)]. 10 ug of the specified DNAs and 1 ug of the plasmid pSV2neo were co-precipitated and transfected onto the packaging cells by standard calcium phosphate transfection procedures. Stably transfected clones were isolated after growth for 14 days in selective media containing 800 ug/ml G418. 24 hour culture supernatants were obtained from confluent monolayers of individual clones and used to infect NIH 3T3 cells. The culture supernatants were removed after 24 hours exposure, and the 3T3 cells were refed with normal media and allowed to grow for an additional 72 hours. Fresh media was placed on these cells for 6 hours and these supernatants were assayed for human tPA with a commercially available ELISA specific for human tPA (Immunobind-5, American Diagnostica Inc., N. Y., N.Y.) From this screen, clones of the packaging cell line producing the MFG-tPA recombinant virus were selected and designated MFG 68.

Canine endothelial cells were isolated from 10 cm segments of the external jugular vein by collagenase digestion as described [T.J. Hunter, S.P. Schmidt, W.V. Sharp, (1983) Trans. Am. Soc. Artif. Intern. Organs 29:177]. The cells were propagated on fibronectin-coated tissue culture dishes in M199 media containing 5% plasma-derived equine serum, 50 ug/ml endothelial cell growth factor, and 100 ug/ml heparin. Purity of the cell cultures was determined by immunohistochemical assay for the presence of Von Willebrands Factor and the absence of smooth muscle cell specific α-actin. The day before transduction, the endothelial cells were seeded at 5.5 X 10³ cells/cm² in medium without heparin. The following day, the endothelial cells were exposed for 24 hours to supernatants containing recombinant virus derived from each producer cell line to which was added 8 ug/ml polybrene. The viral supernatants were removed, the cells fed with normal media and growth was allowed to proceed for an additional 48 hours before analysis.

High molecular weight genomic DNA and total RNA were isolated from cultures of endothelial cells by standard techniques (Molecular Cloning-A Laboratory Manual, T. Maniatis, E.F. Fritsch, and J. Sambrook). The DNA and RNA were analyzed by hybridization analysis with a ³²P-labeled DNA probe prepared from the entire tPA cDNA fragment. Standard techniques were used for electrophoretic separation, filter transfer, hybridization, washing, and ³²P-labeling (Molecular Cloning-A Laboratory Manual T. Maniatis, E.F. Fritsch, and J. Sambrook) . The production of human tPA in transduced canine endothelial cells was demonstrated with a species specific immunocytochemical stain. Transduced cells were fixed in 3% formaldehyde for 10 minutes at room temperature and then permeabilized in 0.1% Triton X-100 for 5 minutes. The fixed cell monolayer was then incubated sequentially with a murine monoclonal antibody to human tPA, with an alkaline phophatase conjugated goat anti-mouse antibody, and finally with a color reagent specific for alkaline phophatase. This procedure specifically stains those cells expressing human tPA and can be visualized by conventional light microscopy. In addition, tPA secretion from transduced cells was determined from confluent cell monolayers. Fresh media was placed on the cells for 6 hours, removed and clarified by centrifugation, and the amount of human tPA determined with a commercially available ELISA (immunobind-5, American Diagnostica).

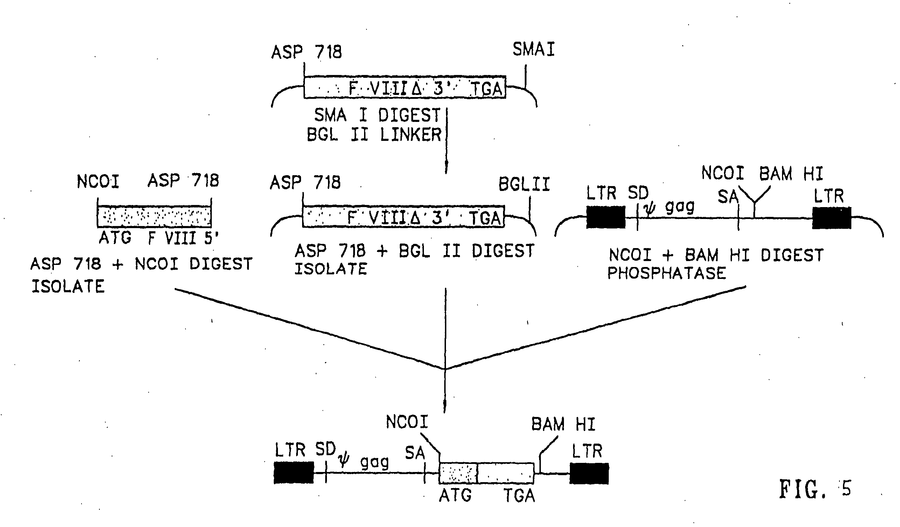

The efficiency of the transduction process is shown by immunocytochemical stain of a population of cells mock transduced or transduced with MFG-tPA. As shown in Figure 5, after a single exposure of the cells to a viral supernatant harvested from MFG 68, essentially all of the cells are synthesizing human tPA as opposed to none of the cells in the control. This was achieved without selection of any type for transduced cells.

An immunological assay was conducted to determine the amount of tPA that was being secreted from transduced cultures. As shown below, cells transduced with recombinant virus from either MFG 68 secreted large amounts of human tPA. Under similar conditions, human endothelial cells in culture typically secrete approximately 1 ng of tPA [Hanss, M., and D. Collen (1987) J. Lab, Clin. Med, 109: 97-104].

**TABLE I**

| Cells | ng human |
|---|---|
| tPA/million | |
| cells/6 hours | |
| uninfected K9 EC | 0.0 |
| MFG 68 K9 EC | 150.1 |

As a further confirmation that the endothelial cells had been transduced with recombinant virus from MFG 68 DNA and RNA was isolated from transduced cells and analyzed by hybridization to a radiolabeled tPA gene. An autoradiogram of the DNA analysis was performed. No hybridization was detected in the uninfected controls, but single hybridizing species of the appropriate molecular weight was seen in the cells infected with the two recombinant vectors. This demonstrates that the genetic information has been transferred to the genome of these transduced cells.

Hybridization analysis of total RNA isolated from these cells confirms the protein and DNA results. Again no hybridization was detected in the control cells but in the RNA derived from the transduced cells hybridizing bands of the appropriate sizes can be seen. RNA from the MFG 68 recombinant virus producing cells is also shown as controls.

### EXAMPLE V In vivo Function of Transduced Canine Endothelial Cells Transplanted on the Surface of Vascular Grafts

Endothelial cells were enzymatically harvested from external jugular veins of adult female mongrel dogs that weighed 20-25 kg and cultured in the laboratory and analyzed for purity as described in Example IV. One half of the cells isolated from each animal were transduced by two exposures to supernatants harvested from the MFG 68 cell line producing the MFG-tPA recombinant virus as described in the previous section. The other half were mock transduced. Growth curves conducted on each population showed no difference in growth characteristics. ELISA measurements were made on culture supernatants derived from each batch of transduced cells to assure that tPA was being secreted from the augmented cells. These cells were then propagated in the laboratory for approximately one week to obtain sufficient numbers of cells.

For each animal from which cells had been isolated, two vascular grafts made of expanded Teflon (W.L. Gore and Associates, Inc. Flagstaff, AZ) were seeded with cells. One graft was seeded with mock transduced cells, and the other with cells transduced to secrete high levels of tPA. Each graft, measuring 0.4 cm x 14 cm, was precoated with 1.5 ug/cm² fibronectin (Sigma Chemical Corp., St. Louis MO), and then seeded with 2200,000 endothelial cells/cm. The grafts were then incubated for an additional 72 hours in culture. Prior to implant the ends were cut off each graft and checked to assure cell coverage.

The same dogs from which the cells had been harvested were anesthetized and 10 cm segments of the seeded grafts were implanted as aorta-iliac bypasses. Each dog received two contralateral grafts; one seeded with control cells and the other seeded with cells that had been transduced to secrete high levels of tPA. Following implantation the performance of the grafts was monitored daily with a B-mode scanner which locates the graft with ultrasound and assesses blood flow through the graft by Doppler measurements (Accuson, Inc.). No drugs to reduce thrombus formation were administered to the animals.

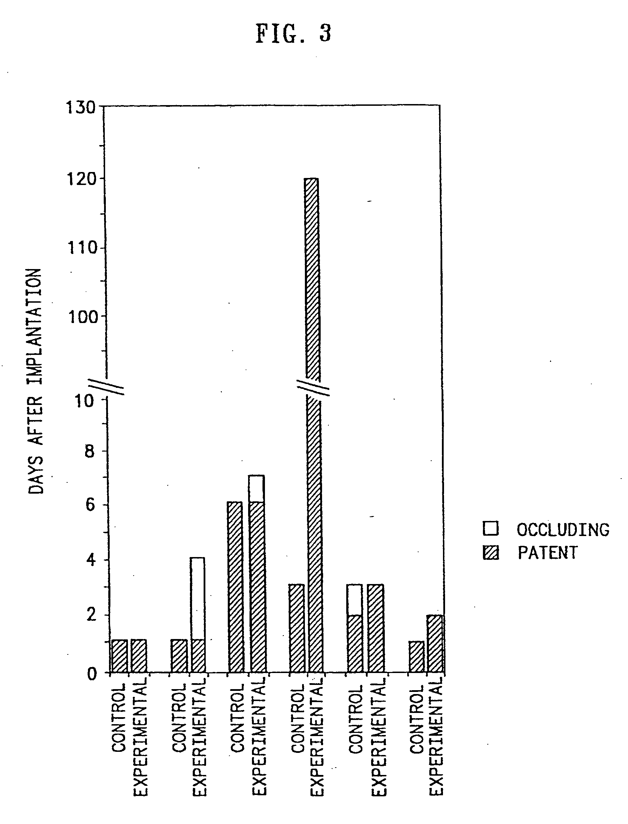

The results of graft performance in 6 different animals were analyzed. The results are indicated in Figure 3. The implant model described above is an extremely stringent one and leads to rapid graft failure by occlusive clot formation. Normal graft function is denoted by solid bar, and a graft which is failing but still functioning by a striped bar. In the first animal, the control graft and the graft lined with transduced cells secreting enhanced levels of tPA (experimental) failed due to clot formation 24 hours after implant. In all of the other five animals, the graft lined with transduced cells secreting enhanced levels of tPA functioned longer than the graft with cells which had only been mock transduced. This difference varied from 24 hours to several months. These results demonstrate that a therapeutic effect can be achieved in vivo with transduced endothelial cells.

### EXAMPLE VI Production of Human Factor VIII from Transduced Endothelial Cells

Endothelial cells were genetically augmented to produce human factor VIII by transducing cells with a retroviral vector, MFG, containing a modifiedhuman factor VIII gene (ATCC accession no. 68726). The modified factor VIII cDNA contains all of the coding sequences for the A1, A2, A3, C1, and C2 domains, however the B domain is deleted from amino acids 743 to 1648. The removal of the B domain and the insertion of the modified factor VIII gene into the retroviral vector MFG is described in detail below and depicted in Figure 5.

Afull-length cDNA without the 5' and 3' untranslated sequences was obtained in a plasmid vector inserted between the restriction sites Nco I (5') and Xho I (3'). For removal of the B domain, the factor VIII cDNA was subcloned into a plasmid vector in 4 fragments spanning the sequences on both the 5' and 3' sides of the B domain. The first fragment of the factor VIII cDNA was subcloned between the restriction sites Sal I and Pst I in the plasmid vector pUC 9. The plasmid vector was cut with Sal I and Pst I and the 5' phosphates were removed using calf intestinal phosphatase. A 1591 base pair Xho I (nucleotide 7263) to Nde I (nucleotide 5672) fragment, and a 359 base pair Nde I (nucleotide 5672) to Pst I (nucleotide 5313) fragment from the full-length cDNA were isolated and ligated with the Sal I/Pst I digested plasmid vector.

To remove the majority of the sequences encoding the B domain which joins amino acids 742 to 1649 in the same translational reading frame, 4 oligonucleotides were synthesized with a 5' Hind III site and a 3' Pst I site covering 168 base pairs. The oligonucleotides extend from the Hind III site at nucleotide 2427 which encodes amino acid 742 followed by amino acid 1649 which is the first amino acid of the activation peptide of the light chain through to the Pst I site at nucleotide 5313. The plasmid vector pUC 9 was digested with the restriction enzymes Hind III and Pst I, and the 5' phosphates were removed using calf intestinal phosphatase. The oligonucleotides were synthesized as 4 separate strands, kinased, annealed and ligated between the Hind III site and the Pst I site of the plasmid vector.

The subcloned Hind III/Pst I oligonucleotide was juxtaposed to the Pst I/ Xho I fragments in a plasmid vector pUC F8. To generate this plasmid, a new polylink er was inserted into a pUC 9 plasmid backbone with the new polylinker encoding the restriction enzyme sites 5' Sma I-Bam HI-Xho I-Pst I-Hind III-Asp 718-Nco I-Hpa I 3' used. The plasmid vector was digested with the restriction enzymes Bam HI and Hind III, and the 5' phosphates were removed with calf intestinal phosphatase. A partial Pst I/ Bam HI digest of the Pst I/Xho I subclone was used to isolate the 3' terminal factor VIII fragment, and a Pst I/Hind III digest of the subcloned oligonucleotides was used to isolate the heavy and light chain junction fragment. They were ligated into the plasmid vector pUC F8 between the BamHI and Hind III sites.

This subclone containing the factor VIII sequences between nucleotides 2427 and 7205 was digested with Asp 718 and Hind III, and the 5' phosphates were removed using calf intestinal phosphatase. A fragment encoding factor VIII between the restriction enzyme sites Asp 718 (nucleotide 1961) and Hind III (nucleotide 2427) was isolated and ligated into the plasmid vector to generate a subclone (pF8 3' delta) containing the factor VIII sequences from nucleotide 1961 through to the translational stop codon at nucleotide 7205.

The construction of the retroviral vector containing the modified factor VIII gene was carried out by inserting the factor VIII gene between the restriction sites Nco I and Bam HI of the retroviral vector MFG. The factor VIII subclone pF8 3' delta was digested with Sma I and converted to a BglII site using an oligonucleotide linker. An Asp 718/Bgl II fragment was isolated from the 3' factor VIII subclone, and a 5' factor VIII fragment containing the ATG for initiation of translation was isolated as an Nco I (nucleotide 151)/Asp 718 fragment (nucleotide 1961). The retroviral vector MFG was digested with Nco I and Bam HI, and the 5' phosphates were removed using calf intestinal phosphatase. The factor VIII fragments were ligated into the retroviral vector yielding the final factor VIII retroviral construct, see Figure 5.

The cell line producing the retroviral particles was generated by transfection of the retroviral vector MFG/factor VIII into equal numbers of ecotropic packaging cells Psi CRE and amphotropic packaging cells CRIP as described by Bestwick et al. (Proc. Natl. Acad. Sci. USA 85:5404-5408 (1988)). To monitor the extent of superinfection taking place between the 2 host ranges of packaging cells, the production of biologically active factor VIII was measured using the Kabi Diagnostica Coatest for Factor VIII, Helena Laboratories, Beaumont, Texas and the production of viral RNA was measured by an RNA dot blot analysis. At 21 days post transfection, the mixture of transfected packaging cells was co-cultivated with the amphotropic packaging cell line Psi CRIP-HIS. The CRIP HIS packaging cell line is a variant of the previously described CRIP packaging cell line. The CRIP HIS packaging cell line is identical to the Psi CRIP packaging cell line except that the retroviral envelop gene was introduced into the cell by cotransfection with pSV2-HIS plasmid DNA, a different dominant selectable marker gene. The packaging cell lines were cultured at a 1:1 ratio for isolation of a homogeneous amphotropic retroviral stock of transducing particles. The superinfection of the amphotropic packaging cell line CRIP HIS has led to the generation of a stable cell line, HIS 19, which produces recombinant retrovirus that efficiently transduce the modified human factor VIII gene. Antibiotic selection of the retroviral producing cell line was not required to isolate a cell line which produces high-titer recombinant retrovirus. The genomic DNA of the cell line has been characterized by Southern blot hybridization analysis to determine the number of integrated copies of the retroviral vector present in the producer cell line. The copy number in the retrovirus producing cell line is approximately 0.5, therefore on average 50% of the CRIP-HIS packaging cells contain a copy of the retroviral vector with the modified factor VIII gene. The retroviral vector and the modified factor VIII gene are intact without any deletions or rearrangements of the DNA in the packaging cell line. The copy number of the retroviral vector remains constant with the continuous passage of the retrovirus producing cell line. For obtaining the highest titer of recombinant retrovirus, HIS 19 was carried 3 passages in selective histidine minus media followed by 4 passages in completed DMEM media. For the generation of retroviral particles, HIS 19 was seeded at 5x10⁵ - 1x10⁶ cells in a 10 cm cell culture dish. At 48 hours postseeding, approximately 70% confluency, fresh medium (DMEM + 10% calf serum) was added to the plates for collection 24 hours later as the source of recombinant retrovirus for transduction.

The modified factor VIII gene was transduced into canine endothelial cells isolated from the jugular vein. The endothelial cells were seeded at 3x10⁵ cells per 10 cm. dish in complete M199 medium with 5% plasma derived serum (Equine), 100ug/ml heparin, and 50ug/ml endothelial cell growth factor for 4-6 hours. The cells were then incubated overnight in M199 medium with 5% plasma derived serum, and 100ug/ml endothelial cell growth factor overnight without heparin which adversely affects the efficiency of the transduction process. Cells were exposed to the fresh viral supernatant plus polybrene (8 ug/ml) for 24 hours. After removal of the viral supernatant, the cells were put into M199 medium with 5% plasma derived serum, 100 ug/ml endothelial cell growth factor to grow to approximately 70-80% confluence. At that time, the medium was changed to M199 medium with 5% heat inactivated fetal bovine serum (heated at 66°C for 2 hours), and 50 ug/ml of ECGF. Following a 24 hr incubation, the medium was collected and assayed for biological active factor VIII by the Kabi Coatest.

With this retrovirus producing cell line, between 50% and 75% of the endothelial cells were transduced as determined by Southern blot analysis. The factor VIII gene can be transduced at this frequency with a single exposure to the recombinant retrovirus, and without antibiotic selection of the transduced cells. The transduced endothelial cells contain an intact copy of the recombinant retroviral genome and the modified factor VIII gene without any deletions or rearrangements. The rate of production of biologically active factor VIII from the genetically augmented endothelial cells was 400ng/5x10⁶ cells/24 hrs.

## Claims

1. A retroviral vector which is MFG having the identifying characteristics of ATCC 68,754.

2. A retroviral vector according to claim 1, said vector further comprising a gene for expression inserted into its insertion site.

3. A retroviral vector according to claim 2, wherein said gene is selected from an enzyme, a receptor, a drug, factor VIII and tPA.

4. A packaging cell line wherein said cell line has been transfected with a retroviral vector according to any of the preceding claims.

5. A cell which has been transduced with retroviral particles obtained from the packaging cell line of claim 4 wherein said cell is selected from the group consisting of: epithelial cells, fibroblasts, hepatocytes, endothelial cells, myoblasts, astrocytes, lymphocytes, and mesenterial cells.

## Patentansprüche

1. Ein retroviraler Vektor MFG mit den Identifikationscharakteristiken von ATCC 68.754.

2. Ein retroviraler Vektor gemäß Anspruch 1, wobei der Vektor ferner ein in eine Insertionsstelle inseriertes Gen zur Expression umfasst.

3. Ein retroviraler Vektor gemäß Anspruch 2, worin das Gen gewählt ist aus einem Enzym, einem Rezeptor, einem Medikament, Faktor VIII und tPA.

4. Eine Verpackungszelllinie, worin die Zelllinie mit einem retroviralen Vektor gemäß einem der vorhergehenden Ansprüche transfiziert worden ist.

5. Eine Zelle, transduziert mit retroviralen Patrikeln, erhalten aus der Verpackungszelllinie gemäß Anspruch 4, worin die Zelle aus der Gruppe gewählt ist, die aus Epithelzellen, Fibroblasten, Hepatocyten, Endothelzellen, Myoblasten, Astrocyten, Lymphocyten und Mesenterialzellen besteht.

## Revendications

1. Vecteur rétroviral qui est MEG ayant les caractéristiques d'identification de l'ATCC 68 754.

2. Vecteur rétroviral selon la revendication 1, ledit vecteur comprenant en outre un gène pour expression inséré dans ledit site d'insertion.

3. Vecteur rétroviral selon la revendication 2, où ledit gène est choisi parmi celui d'une enzyme, d'un récepteur, d'une drogue, du facteur VIII et du tPA.

4. Lignée cellulaire d'empaquetage où ladite lignée cellulaire a été transfectée avec un vecteur rétroviral selon l'une quelconque des revendications précédentes.

5. Cellule qui a été transduitée avec des particules rétrovirales obtenues de la ligne cellulaire d'empaquetage selon la revendication 4, où ladite cellule est choisie au sein du groupe constitué par : les cellules épithéliales, les fibroblastes, les hépatocytes, les cellules endothéliales, les myoblastes, les astrocytes, les lymphocytes, et les cellules mésentériales.
